# EUROPEAN PATENT APPLICATION

(11) **EP 3 278 780 A1**
(43) Date of publication of application: **07.02.2018**
(21) Application number: 17179509.9
(22) Date of filing: 04.07.2017
(51) Int. Cl.: A61F 13/49, A61F 13/15, B32B 27/12, B32B 37/06, B32B 37/14, B32B 3/26, B32B 3/28, B32B 5/04, B32B 7/04, B32B 38/00

(54) **A METHOD FOR PRODUCING A COMPOSITE ELASTIC TAPE**

(30) Priority: 02.08.2016 IT 201600080979
(71) Applicant: Fameccanica.Data S.p.A., 65129 Pescara (IT)
(72) Inventor: BONELLI, Guido, I-65129 Pescara (IT)
(74) Representative: Marchitelli, Mauro

(57) **Abstract**

A method for producing a composite elastic tape (10), comprising the steps of:
- providing an elastic material (12) having a maximum elastic elongation (Eₘₐₓ), in a direction (Y), of between 600% and 1500%, preferably between 700% and 1200%, more preferably between 800% and 1000%,
- elastically stretching said elastic material (12) in said direction (Y) by an elongation value between 10% and 50%, preferably between 10% and 30% of the maximum elastic elongation (Eₘₐₓ),
- providing at least one sheet of fibrous material (14, 16),
- attaching said elastic material (12), elongated by said elongation value, to said at least one sheet of fibrous material (14, 16) in an undeformed state.

## Description

### Field of the invention

The present invention relates to a method for producing a composite elastic tape that is elastically extendable along at least one direction, for example, perpendicular to the longitudinal axis of the web.

The present invention has been particularly developed for producing composite elastic tapes intended to be applied to the waist region, for example for forming side panels of disposable absorbent sanitary articles. The scope of the invention is not, however, limited to this possible field of application.

### Description of the prior art

Disposable absorbent sanitary articles wearable in the form of pants, such as diapers for babies or absorbent sanitary articles for adults, are often obtained from two continuous composite tapes, spaced apart from each other in a transverse direction, and which advance in a longitudinal direction, between which absorbent cores are attached, arranged transversely with respect to the longitudinal direction. The composite tapes form the front and rear waist regions of the absorbent sanitary articles. The waist regions are often provided with elastic webs in order to give the absorbent product better fit to the wearer's body.

A consolidated technique for producing composite elastic tapes for absorbent sanitary products is to advance- in a longitudinal direction- a continuous elastic material in an elastically elongated state, for example, in a transverse direction to the direction of travel, and to apply two non-woven webs on opposite sides of the elastic material. The sheets of non-woven webs and the elastic material are attached together with a welding pattern or by means of adhesive. When the tension that retains the elastic material in an elongated state is released, the non-woven webs curl up and assume a pleated shape. Normally, this manufacturing technique involves the elongation of the elastic material, prior to attaching the non-woven webs, by a selected elongation value. Typically, the elastic material is stretched up to elongation values, for example, of 200%. One example of this technique for producing composite elastic tapes is described in EP 1 355 604 B1 by the same Applicant, which describes a method that involves: selecting a first and a second material, which can be welded together by ultrasonic welding, selecting an elastic material and placing it between the first and the second material to form a stratified structure, joining together the first and the second material by ultrasonic welding, with the elastic material comprised between them at a plurality of welding points.

The main disadvantage of this technique is that the elastic material, when stretched to high elongation values, for example, close to maximum elongation, is subject to permanent yielding and deformation, so that when the tension that produces the elongation of the material ceases, the elastic material does not return to the original undeformed condition (hysteresis). This results in a loss of elastic elongation capacity of the material and a deterioration of its elastic properties.

According to an alternative known technique, the elastic material is joined by means of adhesive or, alternatively, at welding points to two sheets of non-woven fabric while in a relaxed state, i.e. not elastically elongated with respect to its resting state. The composite elastic tape thus formed must be subsequently subjected to a step of mechanical activation in order to obtain the elastic extensibility characteristics. This mechanical activation step typically consists of stretching in a transverse direction, which causes permanent deformation of the two sheets of non-woven fabric. The subsequent return of the elastic material to the resting condition typically forms a crinkling of the two sheets of non-woven fabric, at least at the portions of the sheets of non-woven fabric not welded together, for example, between the welding points, due to the fact that the two sheets of non-woven fabric have undergone a permanent elongation deformation in the stretching direction. After the step of mechanical activation, the composite elastic tape is able to elastically extend in the direction of stretching. This mechanical activation step can be carried out along the production line or at a later stage. For example, the mechanical activation step could be carried out by the user by subjecting the composite elastic tape (already applied to the absorbent sanitary article) to stretching. The disadvantage of this second technique is that the maximum elastic elongation capacity of the composites thus obtained can generally not exceed 250%, and beyond this value, the non-woven fabric deformation is such that it may lose its own integrity.

### Object and summary of the invention

The object of the present invention is to provide a method for producing an elastically extendable composite elastic tape, which is not affected by the problems of the prior art.

According to the present invention, this object is achieved by a method having the characteristics forming the subject of claim 1.

The claims form an integral part of the disclosure provided here in relation to the invention.

### Brief description of the drawings

The present invention will now be described in detail with reference to the attached drawings, given purely by way of non-limiting example, wherein:
- Figure 1 is a schematic cross-sectional view of a composite elastic tape in a relaxed state, and
- Figure 2 is a schematic perspective view illustrating an apparatus for producing the composite elastic tape illustrated in Figure 1.

### Detailed description

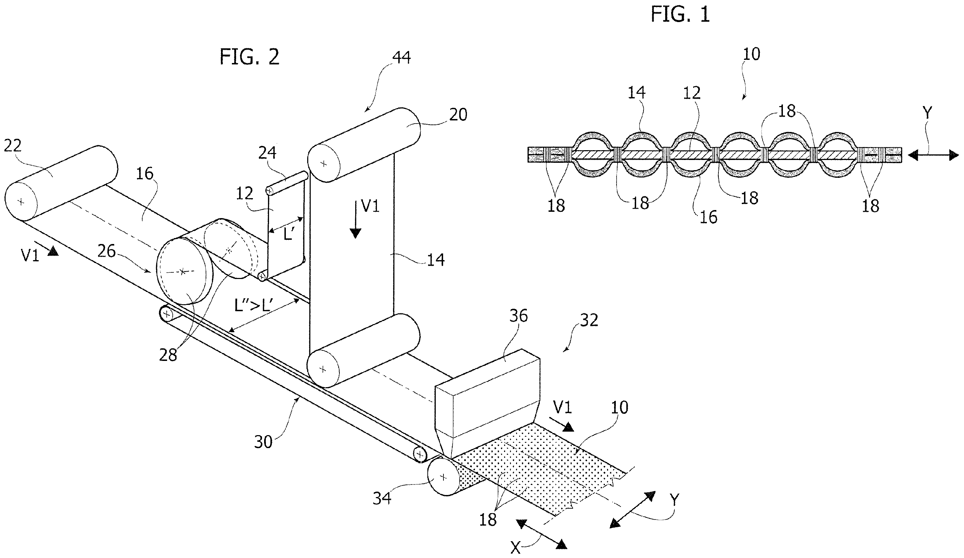

With reference to Figure 1, numeral 10 indicates a composite elastic tape elastically extendable along a transverse direction Y. The composite elastic tape 10 comprises an elastic material 12 elongated along the transverse Y direction between two fibrous sheets 14, 16. The fibrous sheets 14, 16 and the elastic material 12 are attached to each other by means of a plurality of welding points 18. Welding can be carried out via thermal or ultrasonic welding. Alternatively, the sheets forming the composite elastic tape 10 can be glued together by adhesive. The welding points 18 are spaced apart in the longitudinal direction X and in the transverse direction Y to form a welding pattern. The welding points 18 can form openings so as to form breathable passages in the composite elastic tape 10, as described, for example, in the document EP 1 355 604 by the same Applicant.

In general, it can be said that an ultrasonic welding level sufficient to weld together the two fibrous sheets 14, 16 is also such as to provide openings in the elastic material 12 interposed between them. In this way, the first and the second fibrous sheets 14, 16 are welded together through openings that trap the intermediate elastic material 12, by producing a firm anchoring of the composite elastic tape 10 and also creating breathable passages. Ultrasonic welding systems for obtaining a composite elastic tape with breathable openings are described in EP 1 355 604 and in IT 102016000032926 by the same Applicant.

The first and the second fibrous sheets 14, 16 can be made of materials, known in the field of disposable absorbent sanitary articles, which are permeable and generally weldable to each other, typically by ultrasonic welding. Typical materials can be non-woven fibrous materials consisting of synthetic fibers, for example, polyethylene, polypropylene, polyester, bicomponents or combinations of fibers thereof, also in combination with natural fibers such as cotton, rayon, or viscose. For example, the fibrous sheets 14, 16 can be made of non-woven carded or staple fabrics or spunbonds and can be used for the first and second fibrous sheets 14, 16.

In the method according to the present invention, the fibrous sheets 14, 16 selected are those able to have a permanent elongation greater than 200% when subjected to a load of 10 N/50 mm in the required direction, typically transverse, as determined by the EDANA method n. WSP 110.4 (09) option B. Specifically, in the embodiment shown in Figure 1, the fibrous sheets 14, 16 are extensible transversely with respect to the machine direction.

For example, transversely extensible non-woven webs can be used, such as Sawatex 11311D/52 or Sawasoft 2628 produced by Sandler AG, or SoftLite SAP 12.015.005 produced by Jacob Holm.

The elastic material 12 can be formed by a film or by a plurality of webs spaced apart in the direction essentially orthogonal to the extension direction, for example, in the longitudinal direction for an elastic material extensible transversely, as in the embodiment of Figure 2. By way of example, an elastic material transversely extensible and formed of a series of discrete webs, could be produced with the apparatus described in EP 2260813 B1. The elastic material 12 can be selected from the elastic materials known in the field of disposable absorbent sanitary articles. Suitable materials can be films based on thermoplastic elastomeric polymers, for example, elastic polyolefins (PE, PP), styrene block copolymers (SIS, SBS, SEBS), or thermoplastic polyurethanes. In the application of a method according to the present invention, elastic materials are preferably selected having a maximum elastic elongation between 600% and 1500%, preferably between 700% and 1200%, more preferably between 800% and 1000%, determined according to the ASTM D-882 method.

For example, elastic films can be used produced by Tredegar Film Products Italia s.r.l. with codes x36433 and x38495, having a maximum elastic elongation in the transverse direction greater than 900%.

In the manufacturing method of the composite elastic tape 10, the first and the second fibrous sheets 14, 16 and the elastic material 12 are provided in the form of continuous webs collected in reels.

Figure 2 schematically illustrates the steps of a method for producing the composite elastic tape 10. The first fibrous sheet 14 and the second fibrous sheet 16 are fed from respective reels 20, 22 at the same speed V1. The continuous elastic material 12 is fed from a reel 24. The continuous elastic material 12 is fed in the longitudinal direction and is subsequently tensioned in the direction transverse to the selected direction, for example, as shown in Figure 2 in the transverse direction Y perpendicular to the advancing direction of the continuous elastic material 12. The tensioning of the elastic material 12 is between 10% and 50%, preferably between 10% and 30% of the maximum elastic elongation. For example, if the maximum elastic elongation of the material is in the order of 900%, the elastic material 12 is advanced in a transversely elongated state between 100% and 300% relative to the relaxed condition.

The transverse tensioning of the continuous or striped elastic material can be obtained by known apparatuses, for example, by means of a tensioning apparatus 26 of the type described in WO2009/133508, comprising two disks 28 with their respective rotation axes inclined with respect to the transverse Y direction. The tensioning apparatus 28 extends the elastic material 12 in the transverse direction Y from a width L' to a width L". Downstream of the tensioning apparatus 26, the elastic material 12 is kept under tension by a suction conveyor belt 30.

The continuous elastic material 12, in an elastically elongated state, is positioned between the first and the second fibrous sheet 14, 16 while they advance in the direction A in an undeformed state. The stratified structure formed by the two fibrous sheets 14, 16 and by the continuous elastic material 12 is sent to a welding unit 32 comprising an anvil roller 34 and an ultrasonic welding head 36 cooperating with the anvil roller 34. The anvil roller 34 has a cylindrical contrast surface provided with protruding teeth having head surfaces that form counter-welding surfaces cooperating with the ultrasonic welding head sonotrode 36. The protruding teeth of the anvil roller 34 can be aligned in parallel transverse arrays, offset from each other. The welding head 36 joins together the components of the stratified structure by ultrasonic welding of the first fibrous material 14 and the second fibrous material 16 on the head surfaces of the teeth protruding from the anvil roller 34. The continuous elastic material 12 is anchored to the two fibrous sheets 14, 16, while the fibrous sheets 14, 16 are undeformed and while the elastic material 12 is in an elongated state with respect to the resting condition. Downstream of the welding unit 32, the composite elastic tape 10 thus formed advances at speed V1.

A mechanical activation step of the composite elastic tape 10 can be provided downstream of the welding unit 21. Any mechanical activation process that causes permanent elongation of the fibrous sheets 14, 16 can be achieved by known methods directly along the production line, by providing a stretching unit after welding, or it can be achieved later, for example, by the user, by stretching the absorbent sanitary article that incorporates the elastic composite tape 10.

Of course, without prejudice to the principle of the invention, the details of construction and the embodiments can be widely varied with respect to those described and illustrated, without thereby departing from the scope of the invention as defined by the claims that follow.

## Claims

1. A method for producing a composite elastic tape (10), comprising the steps of:
- providing an elastic material (12) having a maximum elastic elongation (Eₘₐₓ), in a direction (Y), of between 600% and 1500%, preferably between 700% and 1200%, more preferably between 800% and 1000%,
- elastically stretching said elastic material (12) in said direction (Y) by an elongation value between 10% and 50%, preferably between 10% and 30% of the maximum elastic elongation (Eₘₐₓ),
- providing at least one sheet of fibrous material (14, 16),
- attaching said elastic material (12), elongated by said elongation value, to said at least one sheet of fibrous material (14, 16) in an undeformed state.

2. A method according to claim 1, comprising the step of providing two sheets of fibrous material (14, 16), and attaching said elastic material (12) elongated by said elongation value between said two sheets of fibrous material (14, 16) in an undeformed state.

3. A method according to claim 1, wherein said at least one sheet of fibrous material (14, 16) has a permanent deformation, in said direction (Y), greater than 200% measured according to the EDANA method no. WSP 110.4 (09) option B.

4. A method according to claim 2, wherein both said sheets of fibrous material (14, 16) have a permanent deformation, in said direction (Y), greater than 200% measured according to the EDANA method no. WSP 110.4 (09) option B.

5. A method according to any one of the preceding claims, comprising after the step of attaching the continuous elastic material (12) to said at least one sheet of fibrous material (14, 16), a step of stretching the composite elastic tape (10) in said direction (Y), so as to cause a permanent deformation of said at least one sheet of fibrous material (14, 16) greater than 200%.

6. A method according to any one of the preceding claims, wherein said direction (Y) is a direction transverse to the longitudinal axis (X) of said composite elastic tape (10).
